# NEW EUROPEAN PATENT SPECIFICATION

(11) **EP 0 586 666 B2**
(45) Date of publication and mention of the opposition decision: **07.02.2001**
(45) Mention of the grant of the patent: 21.05.1997
(21) Application number: 93907552.9
(22) Date of filing: 17.03.1993
(51) Int. Cl.: A61M 37/00, A61N 1/30

(54) **USER ACTIVATED IONTOPHORETIC DEVICE**
BENUTZERAKTIVIERTE, IONTOPHORETISCHE VORRICHTUNG
DISPOSITIF D'IONOPHORESE MIS EN OEUVRE PAR LE PATIENT

(30) Priority: 17.03.1992 SE 9200820; 05.11.1992 US 972280
(43) Date of publication of application: 16.03.1994
(73) Proprietor: Becton, Dickinson and Company, Franklin Lakes, New Jersey 07417-1880 (US)
(72) Inventor: EVERS, Hans Christer Arvid, S-151 59 Sodertalje (SE); BROBERG, Bernt Fredrik Julius, S-619 00 Trosa (SE); DE NUZZIO, John D., Chapel Hill, NC 27516 (US); HOKE, Randal A., Cary, NC 27513 (US)
(74) Representative: Lawrence, Peter Robin Broughton
(86) International application number: US9302427
(87) International publication number: WO9318727

(56) References cited:
- EP-A- 0 060 451
- EP-A- 0 249 475
- EP-A- 0 269 246
- EP-A- 0 498 353
- EP-A- 0 528 789
- WO-A-91/15260
- WO-A-91/15261
- WO-A-92/04938
- DE-A- 3 642 931
- DE-A- 3 903 794
- DE-A- 4 040 911
- DE-C- 3 905 050
- DE-C- 3 905 051
- US-A- 4 666 441
- US-A- 4 994 023
- US-A- 5 053 001
- US-A- 5 077 033
- US-A- 5 087 240
- US-A- 5 128 137
- US-A- 5 158 537
- US-A- 5 215 751
- US-A- 5 466 466

## Description

### FIELD OF THE INVENTION

The present invention generally relates to iontophoretic devices for delivering drugs or medicines to patients transdermally, i.e., through the skin, and more specifically relates to an iontophoretic device capable of being activated by the user.

### BACKGROUND OF THE INVENTION

Transdermal drug delivery systems have, in recent years, become an increasingly important means of administering drugs. Such systems offer advantages clearly not achievable by other modes of administration such as avoiding introduction of the drug through the gastrointestinal tract or punctures in the skin to name a few.

Presently, there are two types of transdermal drug delivery systems, i.e., "Passive" and "Active." Passive systems deliver drug through the skin of the user unaided, an example of which would involve the application of a topical anesthetic to provide localized relief, as disclosed in U.S. Patent No. 3,814,095 (Lubens). Active systems on the other hand deliver drug through the skin of the user using iontophoresis, which according to *Stedman's Medical Dictionary,* is defined as "the introduction into the tissues, by means of an electric current, of the ions of a chosen medicament."

Conventional iontophoretic devices, such as those described in U.S. Patent Nos. 4,820,263 (Spevak et al.), 4,927,408 (Haak et al.) and 5,084,008 (Phipps), the disclosures of which are hereby incorporated by reference, for delivering a drug or medicine transdermally through iontophoresis, basically consist of two electrodes -- an anode and a cathode. Usually, electric current is driven from an external supply into the skin at the anode, and back out at the cathode. Accordingly, there has been considerable interest in iontophoresis to perform delivery of drugs for a variety of purposes. Two such examples, involve the use of Novocaine, which is usually injected prior to dental work to relieve pain, and Lidocaine, which is usually applied as a topical, local anesthetic.

However, several disadvantages and limitations have been associated with the use of such devices, including storage stability as a result of the drug not being in a form suitably stable to provide a commercially practical shelf life. Upon storage for extended periods, the therapeutic agents can degrade and become less potent. In addition, such devices have not delivered an efficient dosage of the drug resulting in poor performance and a need for larger amounts of the drug, which upon completion of the application is wasted. Accordingly, such devices have been generally impractical for use on outpatients and in doctor's offices, since the products do not have sufficient shelf life and neither the patient nor the practitioner wishes to wait the required time for the desired effect.

Several of the prior passive type devices have attempted to overcome or minimize one such limitation, i.e., shelf life, by including a "burstable" member to isolate or separate the drug as disclosed in U.S. Patent Nos. 4,911,707 (Heiber et al.) and 4,917,676 (Heiber et al.). However, limitations remain with respect to the use of such devices, particularly when bursting" the member. During this event, the drug would be mixed with the activating solution limiting the dose efficiency of the device.

Another attempt to overcome this problem has included adding the drug to the device prior to use as disclosed, for example, in U.S. Patent No. 4,722,726 (Sanderson et al.), by injecting the drug into a chamber. However, other limitations remain with respect to the use of such devices, particularly when injecting the drug. During this event, the device is difficult to use, especially by persons who are handicapped or infirmed by some disability or limitation. In addition, such devices have not provided an efficient dosage as a result of the presence of competing ions.

Attempts to provide dose efficient devices have included two-compartment configurations, such as those described in the patents to Haak et al. and Phipos, separating the drug to be administered from the electrolytic solution. However, such devices have failed to address the need for long-term stability and shelf-life to prevent degradation of the drug. Also, slow transport and equilibration between the compartments can dilute the drug formulation, thus decreasing the dose efficiency of the device.

Thus, there has been a need for a user activated iontophoretic device, which would eliminate the problems and limitations associated with the prior art devices discussed above, most significant of these problems being associated with storage of the device, i.e., shelf-life, and dose efficiency.

Haak, et al US Patent 5158537 describes an iontophoretic device where the drug and electrolyte reservoirs are in a non-hydrated condition. Liquid is released from pouches to hydrate the drug reservoir and to hydrate the electrolyte reservoir, using liquid-wicking pathways.

### SUMMARY OF THE INVENTION

In contrast to the prior devices discussed above, it has been found that a iontophoretic device particularly suited for use to deliver a high dose efficiency, while providing a commercially suitable shelf-life can be constructed in accordance with the present invention. In addition, the device of the present invention is easily activated by the user to administer the drug. Such users may include the patient as well as doctors, nurses and the like.

The user activated device for delivering at least one medication to an applied area of a patient, such as the skin mucus membrane and the like, of the present invention is defined in claim 1. It includes electrode assembly means for driving a medication into the applied area of the patient to be absorbed by the body of the patient, a first reservoir situated in relation to the electrode assembly means and a second reservoir containing a medication to be delivered to the applied area of the patient and holding means for holding the electrode assembly means, the first reservoir and the second reservoir. The holding means includes first means for maintaining the electrode assembly means in electrical communication with the first reservoir and second means for maintaining the second reservoir separate in relation to the first reservoir prior to activation such as to prevent degradation and dilution of the medication contained in the second reservoir and upon activation the first reservoir and the second reservoir are brought into contact with one another to at least partially hydrate one of the reservoirs, thus increasing the dose efficiency of the device, while permitting electrical conducting contact between the first reservoir and the second reservoir after activation. The medication contained in the second reservoir is maintained in a dry state prior to activation. In addition, the first reservoir may include a second medication to be delivered to the applied area of the patient.

In one embodiment, the first means and the second means are hingedly connected together along a bendable member so that the device may be activated by folding the holding means along the bendable member to bring the first reservoir and the second reservoir into electrical conducting contact with one another.

In another embodiment, the device also includes barrier means for separating the first reservoir from the second reservoir, which may be manipulated to bring the first reservoir and the second reservoir into electrical conducting contact with one another. In this embodiment, the barrier means is adapted to include an upper release surface, a lower release surface and a pull tab extending from the holding means so that the device may be activated by pulling the tab to remove the barrier from the device.

The method of iontophoretically delivering at least one medication through an applied area of a patient such as the skin, mucus membrane or the like, includes exposing a first portion of a device including an electrode assembly and a first reservoir and exposing a second portion of the device including a second reservoir containing a medication to be delivered to the patient separate from the first portion. The first reservoir of the first portion of the device is brought into electrical conducting contact with the second reservoir of the second portion of the device to at least partially hydrate one of the reservoirs and to form a combined portion, with the combined portion of the device applied to an area of the patient to be treated. In addition, current is caused to flow through the device into the applied area to drive the medication into the body of the patient.

In an alternative embodiment, a hydrating solution is applied to the area of the patient prior to activation of the device and application of the device onto the applied area of the patient.

The user activated iontophoretic device for delivering at least one medication through an applied area of a patient, such as the skin, mucus membrane and the like, of the present invention includes a first portion and a second portion. The first portion includes an electrode assembly and a first reservoir, and the second portion indudes a second reservoir. The electrode assembly includes electrode means for driving a medication into the patient to be absorbed by the body of the patient. The first reservoir which is electrically conductive contains an active compound to be delivered to the applied area of the patient, and the second reservoir contains a vasoactive agent to be delivered to the applied area of the patient. In addition, holding means holds the first portion and the second portion separate from one another, with the electrode assembly maintained in electrically communicating relation with the first reservoir, and with the vasoactive agent contained by the second reservoir maintained separate in relation to the first portion prior to activation. In this way, upon activation, the first reservoir and the second reservoir may be brought into electrical conducting contact with one another and at least of the reservoirs is at least partially hydrated.

In addition, upon activation, the vasoactive agent may be dissolved at the interface of the two reservoirs, with the vasoactive agent in contact with the applied area of the patient. Also, the vasoactive agent may be initially in a dry form separated from the active compound with the holding means sealed to keep the first portion intact during storage. Also, the active compound may include a local anaesthetic such as lidocaine, and the vasoactive agent may include a vasoconstricting compound such as adrenaline.

### BRIEF DESCRIPTION OF THE DRAWINGS

The various features, objects, benefits, and advantages of the present invention will become more apparent upon reading the following detailed description of the preferred embodiment along with the appended claims in conjunction with the drawings, wherein like reference numerals identify corresponding components, and:
Figures 1A & 1B are schematic, cross sectional views of one embodiment of the user activated iontophoretic device of the present invention, with Figure 1A illustrating the device prior to activation and Figure 1B illustrating the device after activation;
Figure 2 is a schematic, cross sectional view of another embodiment of the device of the present invention illustrated prior to activation:
Figures 4A & 4B are schematic, cross sectional views of another embodiment of the device of the present invention including a second drug reservoir and illustrated prior to and after activation;
Figure 5 is a schematic, cross sectional view of another embodiment the device illustrated prior to activation; and
Figures 6A, 6B & 6C are schematic views of yet another embodiment of the device of the present invention, with

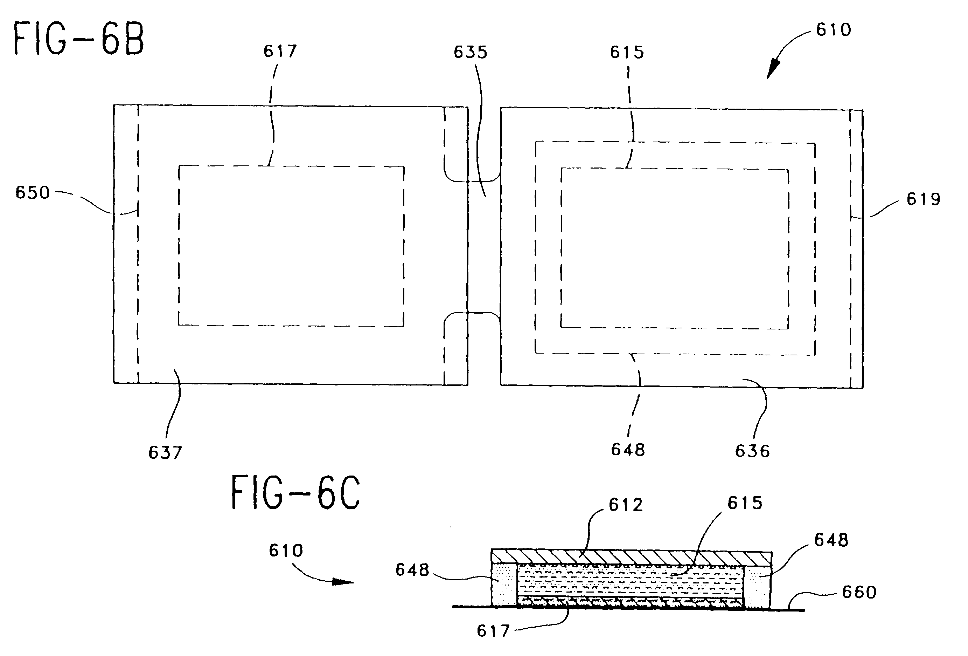

Figure 6A being a cross sectional view illustrating the device prior to activation, Figure 6B being a bottom view illustrating the device prior to activation, and Figure 6C being a cross section side view illustrating the device after activation.

### DETAILED DESCRIPTION OF THE PREFERRED EMBODIMENT

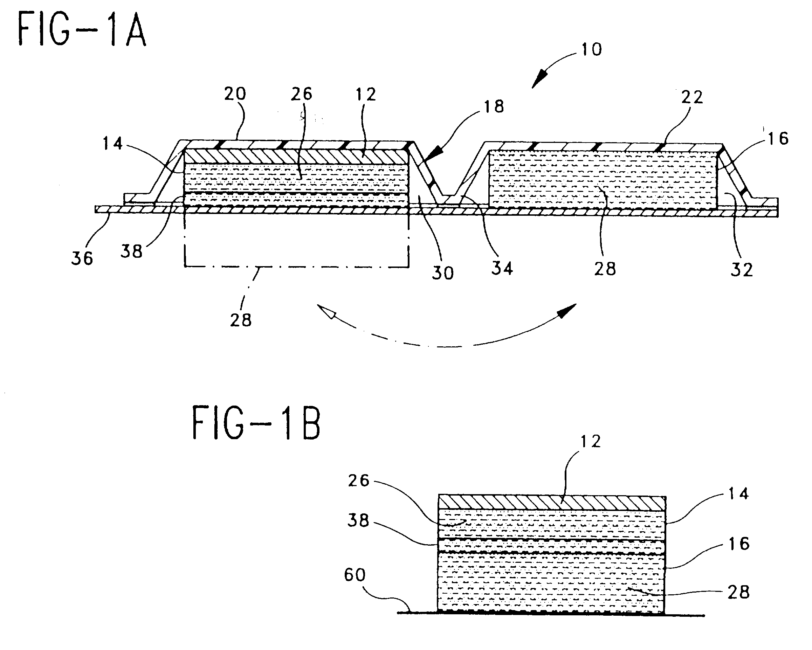

The user activated iontophoretic device of the present invention is illustrated in Figures 1-6 and generally includes the designation 10. Referring to Figures 1A and 1B, the device or patch 10 of the present invention includes an electrode assembly 12, having at least one electrode, an electrode reservoir 14 and at least one drug reservoir 16, which are held or contained within a pouch or other suitable structure 18. It should be appreciated that a return electrode may be combined in the assembly 12 or separately provided as is well known in the art.

In the preferred embodiment, the device is divided or otherwise separated into two portions, one portion 20 (first) includes the electrode assembly 12 and the electrode reservoir 14 with the electrode reservoir being situated adjacent to the electrode assembly and holding an electrolyte 26. The other portion 22 (second) includes the drug reservoir 16 which holds the medication or drug 28, preferably in an ionized or ionizable form, to be delivered iontophoretically. The particular electrolyte is not essential to the present invention and is merely a matter of choice. However, in this embodiment the electrolyte may include sodium chloride in an aqueous solution, gel matrix or the like.

The pouch 18 has at least two compartments 30, 32, with one compartment 30 (first) containing the first portion 20 of the device and the second compartment 32 containing the other portion 22 (second). The two compartments 30, 32 are hingedly connected together along a bendable member 34 with a release liner 36 sealing the two compartments. In this way, the drug can be stored or otherwise isolated from the first portion, in a dry state or formulation in a matrix or on a supporting substrate, which can be hydrated prior to use. Also, the drug can be stored in a non-aqueous solvent such as low molecular weight polyethylene glycol or glycerine. The drug may be stable in such non-aqueous solvents, and the solution (with the ionized or ionizable drug) may be an adequate electrolyte depending upon the particular drug or combination of drugs. These solvents might also be used as humectants in a gel matrix.

At least one barrier 38 may be situated between the electrode reservoir 14 or the drug reservoir 16, either adjacent to the one or the other, to limit transport of ions between the two reservoirs when the two portions are caused to come into electrical conducting contact with one another as illustrated in Figure 1B. The particular barrier used in this embodiment is not essential to the present invention and may include any of the membranes or forms described in the patents to Haak et al. and Phipps, depending upon the particular therapeutic application.

In the preferred embodiment, the two portions are brought into contact with one another by first removing the release liner 36 to expose the two portions 20, 22 and folding or otherwise bending the device along the bendable member to bring the two portions into contact with one another.

As is well known within the field, the device can then be applied to the patient and a voltage impressed across the electrodes of the electrode assembly 12 to cause current to flow through the skin 60 of the patient to drive the ionic medication into the skin and the tissue to be absorbed by the body of the patient. It should also be appreciated that the device of the present invention can be applied to other areas of the body such as mucus membranes depending upon the desired therapy and drugs to be delivered.

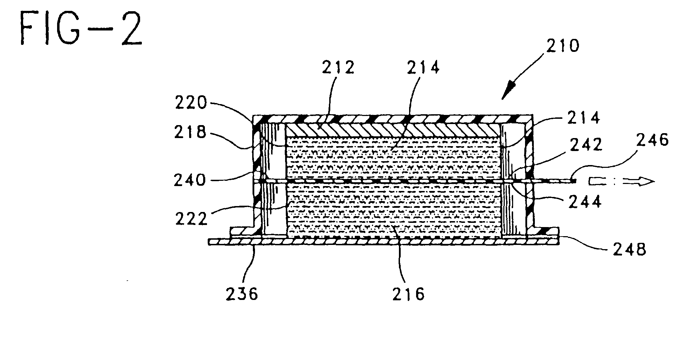

Another embodiment of the device of the present invention is illustrated in Figure 2, and is generally designated 210 with the two portions 220, 222 of the device contained in a single structure 218 and separated by a barrier 240. The barrier 240 includes an upper release surface 242, a lower release surface 244 and a pull tab 246 extending from the structure. The release surfaces are provided to prevent the barrier from adhering to the adjacent portions of the device such as the reservoirs 214, 216.

The device 210 may also include a layer of adhesive 248 for adhering the device to the skin of the patient. However, prior to applying the device to the patient, the barrier 240 is removed by pulling the tab 246 to remove the barrier from the device and cause the electrode reservoir 214 and the drug reservoir 216 to come into electrical conducting contact with one another.

It should be appreciated that other forms of barriers may be used as long as they separate the two reservoirs 214, 216 of the two portions prior to application to prevent degradation of the drug through, for example, slow transport or equilibration between the reservoirs, or through other action which would otherwise result in the drug formulation being diluted, thus decreasing the dose efficiency of the device, while permitting electrical conducting contact between the reservoirs after activation. In this embodiment, the barrier is a vapor/liquid impermeable barrier which may be manipulated by being removed to activate the device.

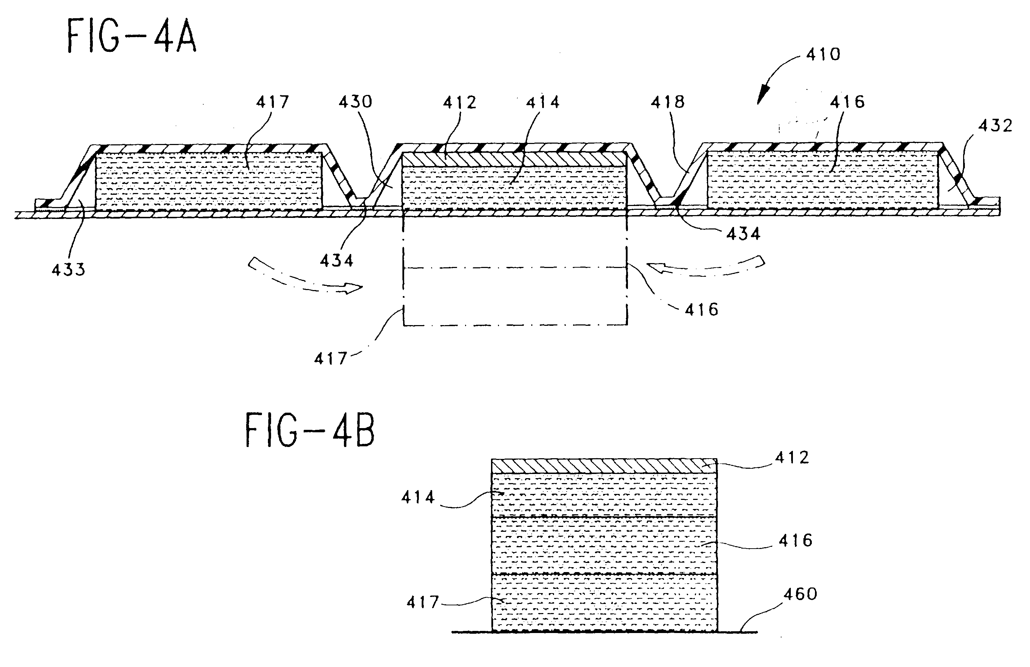

In the embodiment illustrated in Figures 4A and 4B, the device is generally designated 410 and includes a pouch or suitable structure 418 having three compartments 430, 432, 433, each separated by a bendable member 434. The first compartment 430 contains the electrode assembly 412, electrode reservoir 414, the second compartment contains the drug reservoir 416 and the third compartment contains a second drug reservoir 417 which is brought into contact with the skin 460 of the patient.

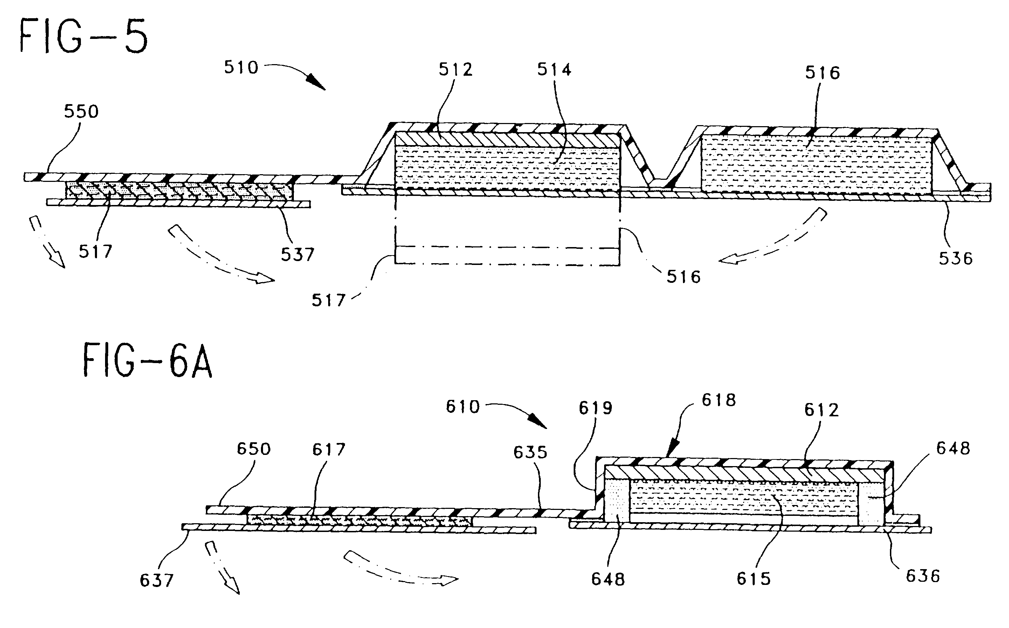

In the alternative, as illustrated in Figure 5, the third portion of the device 510 may include a flexible member 550 upon which the second drug reservoir 517 may be attached. In this embodiment, the flexible member 550 simply includes the existing release liner 536 and a second release liner 537 with the second drug reservoir 517 sandwiched therebetween.

An alternative embodiment is illustrated in Figures 6A, 6B and 6C, and generally designated as 610. The device includes the electrode assembly 612, a first reservoir 615 combining the electrode reservoir and the first drug reservoir, and a second reservoir 617. These elements are contained within the structure 618 and covered by a backing or covering 619.

The first portion consists of the electrode assembly 612 and the first reservoir 615, with the second portion simply consisting of the second reservoir 617. The layer of adhesive 648 surrounds at least a portion of the first reservoir with the release liner 636 covering the exposed surfaces of both.

The second reservoir 617 is attached on one side to the flexible member 650 and on the other side to a second release liner 637, with the second reservoir sandwiched therebetween. The bendable member 635 hingedly connects the flexible member 650 to the backing 619 of the structure 618. In this embodiment, the backing preferably includes a foil material, which, e.g., may be plastic-laminated aluminum.

The first reservoir 615 of the device 610 can be electrically conductive and pre-assembled in electrical conducting contact with the electrode assembly 612, and prior to activation the second reservoir 617 can be brought into electrical conducting contact with the first reservoir along their interface. In this way, the first reservoir 615 can be used to contain a gel with an active compound such a local anaesthetic, e.g., lidocaine, dispersed therein, and the second reservoir 617 can be used to contain a vasoactive agent such as adrenaline. The addition of the vasoactive agent provides additional localization of the local anaesthetic agent at the applied area by being vasoconstricting, which has been found to significantly increase both the depth or magnitude of dermal analgesia and prolongation of the duration of the desired effect.

Upon activation, the vasoactive agent may be dissolved at the interface of the reservoirs, due to its solubility in an aqueous fluid. Thus, after attaching the device to a suitable area of the skin 660 of the patient, with the vasoactive agent in contact with the skin, current can be applied. The local anaesthetic agent and the active compound act together during the iontophoretic administration period.

Due to oxidation and hydrolysis of the in aqueous solutions instable substances, such as adrenaline, during storage at normal, that is room temperatures, it has been found that the activity of the vasoactive substance as a vasoconstrictor is decreased, unless special precautions are taken. These procedures, prior hereto, have involved a complete elimination of oxygen in the container for the active agent, and the use of laminar nitrogen flow during the process of filling the container with the combined local anaesthetic-adrenaline solution.

In the preferred embodiment, the vasoactive agent can be kept in a dry form, separated from the local anaesthetic (active compound), with the structure 618 sealed to keep the aqueous solution intact during storage and where the vasoactive agent is kept in its dry form, separated from the local anaesthetic solution during storage. The vasoactive agent is preferably kept in its dry form homogeneously distributed in a carrier material, e.g., cotton fiber, woven plastic thread and the like, with the same surface area as the first reservoir 615 containing the local anaesthetic (active compound).

Adrenaline in dry form, i.e., adrenaline thread which includes a cotton or woven plastic thread, impregnated with adrenaline in a pre-determine amount per unit length has been found to be suitable for use in the device of the present invention. The stability of the dry adrenaline present in this formulation has been found to be extremely favorable even when stored at room temperature for more than five years.

The gel contained in the first reservoir 615 and used for the electrolyte may also act as an adhesive, eliminating the need for the adhesive layer 648. In addition, a porous adhesive may be used.

The following formulations may be used in connection with the embodiment of the device 610 of the present invention illustrated in Figures 6A, 6B and 6C:

### EXAMPLE 1

| | |
|---|---|
| Lidocaine hydrochloride monohydrate corresponding to lidocaine hydrochloride | 150 mg |
| Purified Water | 1 ml |

Lidocaine hydrochloride monohydrate is dissolved in purified water. The solution is adsorbed into a thin material of cellulose or plastic, such as Vetx® or Porex®.

### EXAMPLE 2

| | |
|---|---|
| Ropivacaine hydrochloride monohydrate corresponding to ropivacaine hydrochloride | 35 mg |
| Purified Water | 1 ml |

Ropivacaine hydrochloride monohydrate is dissolved in purified water. The solution is adsorbed into a thin material of cellulose or plastic.

### EXAMPLE 3

| | |
|---|---|
| Lidocaine hydrochloride monohydrate corresponding to lidocaine hydrochloride | 20 mg |
| Purified Water | 1 ml |

The preparation is prepared as with Example 2.

### EXAMPLE 4

| | |
|---|---|
| Adrenaline base | 1.68 g |
| Hydrochloride add | 5.04 ml |
| Sodium pyrosulfite | 0.10 g |
| Disodium tetracemin | 0.05 g |
| Purified Water | 100 g |

By varying the amount of water, solutions with different contents of vasoactive agent are obtained. The carrier material to be soaked with the vasoactive agent is of cotton thread, synthetic fibre or paper. The carrier material is soaked with the vasoactive solution and the solution is evaporated until the carrier is dry.

### EXAMPLE 5

| | |
|---|---|
| Felypressin, Octapressin® stock solution 25 IU/ml | 3.15 IU |
| Purified Water | 100 g |

By varying the amount of water, solutions with different contents of the peptide felypressin are obtained. The carrier material to be soaked with the peptide is of cotton thread, synthetic fiber or paper. The material is soaked with the peptide and the solution is evaporated until the carrier is dry.

In addition, peptides, such as felypressin have been found to be suitable. Preferably, both the active components, the local anaesthetic in its hydrochloride salt form or the peptide, and the vasoconstrictor (preferably adrenaline) are both easily soluble in aqueous solutions.

Thus, the various embodiments of the present invention can be used wherein at least one of the active compounds needs to be isolated. Drug, medication and active compound have been used herein to mean any pharmaceutical agent, such as therapeutic compounds, diagnostic agents and the like.

The particular matrix of the material or the method of manufacture is not essential to the present invention. For example, the drug can be spray-dried onto an inert support such as a non-woven material, a screen or scrim, or a variety of micro-porous supports such as nylon, polyethylene, and polypropylene. In addition, the drug can be dispersed in an ointment or liquid and cast and dried onto a support. Also the drug can be mixed with dispersing agents or watersoluble polymers and pressed into a dry wafer or pellets that dissolve rapidly in water. The drug can be uniformly dispersed in a de-hydrated gel that can be hydrated rapidly from an added source of water.

In addition, while the present invention has been described in connection with iontophoresis, it should be appreciated that it may be used in connection with other principles of active introduction, i.e., motive forces, such as electrophoresis which indudes the movement of particles in an electric field toward one or other electric pole, anode, or cathode and electro-osmosis which includes the transport of uncharged compounds due to the bulk flow of water induced by an electric field. Also, it should be appreciated that the patient may include humans as well as animals.

While the preferred embodiments of the present invention has been described so as to enable one skilled in the art to practice the device of the present invention, it is to be understood that variations and modifications may be employed without departing from the concept and intent of the present invention as defined in the following claims. The preceding description is intended to be exemplary and should not be used to limit the scope of the invention. The scope of the invention should be determined only by reference to the following claims.

## Claims

1. A user activated device (10) delivering at least one medication (28) to an applied area of a patient, comprising:
an electrode assembly means (12) for driving a medication (28) into the applied area of the patient to be absorbed by the body of the patient;
wherein the electrode assembly (12) is situated in electrical communication with a first reservoir (14) which contains a substantially hydrated electrolyte;
a second reservoir (16) containing a substantially non-hydrated medication (28) to be delivered to the applied area of the patient;
a first holding means (20) for holding the electrode assembly means (12) and the first reservoir (14), said first holding means (20) including a means for maintaining the electrode assembly means (12) in electrical communication with the first reservoir (14); a second holding means (22) including a means for holding the second reservoir (16) and including a means for maintaining the second reservoir (16) separate in relation to the first reservoir (14); the first holding means (20) and the second holding means (22) each having a release liner (36,240,336,536,537,636,637) for sealing the first holding means (20) and the second holding means (16) so that prior to activation the second reservoir (16) containing the medication (28) is isolated from the first reservoir (14) and maintained in a non-hydrated condition to prevent degradation and dilution of the medication contained in the second reservoir (16); the device (10) being activatable by removing the release liner (36) and placing the first reservoir (14) in contact with the second reservoir (16), thereby to at least partially hydrate the medication (28) contained in the second reservoir (16) and to bring the first reservoir (14) and the second reservoir (16) into electrical communication with one another, and in which either (a) said first holding means (20) and said second holding means (22) are hingedly connected together along a bendable member (34) so that the device (10) may be activated by folding the holding means (20,22) along the bendable member (34) to bring the first reservoir (14) and the second reservoir (16) into electrical communication or (b) the device comprises barrier means (240) for separating said first reservoir from said second reservoir, which may be manipulated to bring the first reservoir and the second reservoir into electrical conducting contact with one another; and said barrier means (240) is adapted to include an upper release surface (242), a lower release surface (244) and a pull tab (246) extending from the holding means so that the device may be activated by pulling the tab (246) to remove the barrier (240) from the device to bring the first reservoir and the second reservoir into electrical communication.

2. A user activated device as defined in claim 1 wherein said first reservoir indudes a second medication to be delivered to the applied area of the patient, and said second reservoir is maintained in a dry state prior to activation.

3. A user activated device as defined in either preceding claim, further comprising at least one barrier (38) situated between the first reservoir and the second reservoir to limit the presence of competing ions when the two reservoirs are in electrical conducting contact with one another.

4. A user activated device according to claim 1, which is an iontophoretic device wherein said second reservoir contains a substantially non-hydrated vasoactive agent medication to be delivered to the applied area of the patient.

5. A user activated iontophoretic device as defined in claim 4, wherein the first reservoir includes a local anaesthetic and the vasoactive agent is a vasoconstricting compound.

6. A user activated iontophoretic device as defined in claim 5, wherein the local anaesthetic is lidocaine and the vasoconstricting compound is adrenaline.

7. A user activated device as defined in any preceding claim, wherein the electrolyte is an electrically conductive gel.

## Patentansprüche

1. Nutzeraktivierte Vorrichtung (10), die mindestens ein Medikament (28) an einen Applikationsbereich eines Patienten abgibt, mit:
einer Elektrodeneinheit (12), um ein Medikament (28) in einen Applikationsbereich des Patienten zu treiben, damit es vom Körper des Patienten absorbiert wird;
wobei die Elektrodeneinheit (12) in elektrischer Verbindung mit einem ersten Vorratsbehälter (14) steht, der einen im wesentlichen hydratisierten bzw. wasserhaltigen Elektrolyten enthält;
einem zweiten Vorratsbehälter (16), der ein im wesentlichen nichthydratisiertes Medikament (28) enthält, das zum Applikationsbereich des Patienten abgegeben werden soll;
einer ersten Halteeinrichtung (20) zum Festhalten der Elektrodeneinheit (12) und des ersten Vorratsbehälters (14), wobei die erste Halteeinrichtung (20) eine Einrichtung einschließt, um die Elektrodeneinheit (12) in elektrischer Verbindung mit dem ersten Vorratsbehälter (14) zu halten; einer zweiten Halteeinrichtung (22), die eine Einrichtung zum Festhalten des zweiten Vorratsbehälters (16) sowie eine Einrichtung einschließt, die den zweiten Vorratsbehälter (16) vom ersten Vorratsbehälter (14) getrennt hält; wobei die erste Halteeinrichtung (20) und die zweite Halteeinrichtung (22) jeweils eine Trennschicht (36, 240, 336, 536, 537, 636, 637) zum Abdichten der ersten Halteeinrichtung (20) und der zweiten Halteeinrichtung (22) aufweisen, so daß der zweite Vorratsbehälter (16), der das Medikament (28) enthält, vor der Aktivierung vom ersten Vorratsbehälter (14) isoliert ist und in einem nichthydratisierten Zustand gehalten wird, um eine Zersetzung und Verdünnung des im zweiten Vorratsbehälter (16) enthaltenen Medikaments zu verhindern; wobei die Vorrichtung (10) durch Entfernen der Trennschicht (36) und Inkontaktbringen des ersten Vorratsbehälters (14) mit dem zweiten Vorratsbehälter (16) aktivierbar ist, um dadurch das im zweiten Vorratsbehälter (16) enthaltene Medikament (28) zumindest teilweise zu hydratisieren sowie um den ersten Vorratsbehälter (14) und den zweiten Vorratsbehälter (16) in elektrische Verbindung miteinander zu bringen, und wobei entweder
(a) die erste Halteeinrichtung (20) und die zweite Halteeinrichtung (22) längs eines biegefähigen Gliedes (34) gelenkig miteinander verbunden sind, so daß die Vorrichtung (10) durch Zusammenklappen der Halteeinrichtungen (20, 22) längs des biegefähigen Gliedes (34) aktiviert werden kann, um den ersten Vorratsbehälter (14) und den zweiten Vorratsbehälter (16) in elektrische Verbindung miteinander zu bringen, oder
(b) die Vorrichtung eine Sperrschichteinrichtung (240) zum Trennen des ersten Vorratsbehälters vom zweiten Vorratsbehälter umfaßt, die so manipuliert werden kann, daß der erste Vorratsbehälter und der zweite Vorratsbehälter in elektrisch leitenden Kontakt miteinander gebracht werden können; und wobei die Sperrschichteinrichtung (240) so eingerichtet ist, daß sie eine obere Trennfläche (242), eine untere Trennfläche (244) und eine von der Halteeinrichtung ausgehende Zug- bzw. Aufreißlasche (246) aufweist, so daß die Vorrichtung durch Ziehen an der Lasche (246) aktiviert werden kann, um die Sperre (240) von der Vorrichtung zu entfernen und den ersten Vorratsbehälter und den zweiten Vorratsbehälter in elektrische Verbindung miteinander zu bringen.

2. Nutzeraktivierte Vorrichtung nach Anspruch 1, wobei der erste Vorratsbehälter ein zweites Medikament enthält, das an den Applikationsbereich des Patienten abgegeben werden soll, und wobei der zweite Vorratsbehälter vor der Aktivierung in trockenem Zustand gehalten wird.

3. Nutzeraktivierte Vorrichtung nach einem vorhergehenden Anspruch, die ferner mindestens eine Sperre (38) aufweist, die zwischen dem ersten Vorratsbehälter und dem zweiten Vorratsbehälter angeordnet ist, um die Gegenwart konkurrierender lonen zu begrenzen, wenn sich die beiden Vorratsbehälter in elektrisch leitendem Kontakt miteinander befinden.

4. Nutzeraktivierte Vorrichtung nach Anspruch 1, die eine iontophoretische Vorrichtung ist, wobei der zweite Vorratsbehälter ein im wesentlichen nichthydratisiertes vasoaktives Medikament ist, das zum Applikationsbereich des Patienten abgegeben werden soll.

5. Nutzeraktivierte iontophoretische Vorrichtung nach Anspruch 4, wobei der erste Vorratsbehälter ein lokales Anaesthetikum enthält und das vasoaktive Mittel ein Vasokonstriktor ist.

6. Nutzeraktivierte iontophoretische Vorrichtung nach Anspruch 5, wobei das lokale Anaesthetikum Lidokain und der Vasokonstriktor Adrenalin ist.

7. Nutzeraktivierte Vorrichtung nach einem der vorstehenden Ansprüche, wobei der Elektrolyt ein elektrisch leitendes Gel ist.

## Revendications

1. Dispositif activé par un utilisateur (10) délivrant au moins un médicament (28) à un endroit d'application du patient, comprenant:
un moyen d'ensemble d'électrodes (12) pour entraîner un médicament (28) dans l'endroit d'application du patient pour qu'il soit absorbé par le corps du patient ;
dans lequel l'ensemble d'électrodes (12) est situé en communication électrique avec un premier réservoir (14) qui contient un électrolyte substantiellement hydraté ;
un second réservoir (16) contenant un médicament substantiellement non hydraté (28) à délivrer à l'endroit d'application du patient ;
un premier moyen de support (20) pour maintenir le moyen d'ensemble d'électrodes (12) et le premier réservoir (14), ledit premier moyen de support (20) incluant un moyen pour maintenir le moyen d'ensemble d'électrodes (12) en communication électrique avec le premier réservoir (14); un second moyen de support (22) incluant un moyen pour maintenir le second réservoir (16) et incluant un moyen pour maintenir le second réservoir (16) séparé par rapport au premier réservoir (14); le premier moyen de support (20) et le second moyen de support (22) ayant chacun une bande détachable (36) pour sceller le premier moyen de support (20) et le second moyen de support (22) de sorte qu'avant l'activation, le second réservoir (16) contenant le médicament (28) est isolé du premier réservoir (14) et maintenu dans une condition non hydratée pour empêcher la dégradation et la dilution du médicament contenu dans le second réservoir (16); le dispositif (10) pouvant être activé en détachant la bande détachable (36) et en plaçant le premier réservoir (14) en contact avec le second réservoir (16), pour hydrater ainsi au moins partiellement le médicament (28) contenu dans le second réservoir (16) et mettre le premier réservoir (14) et le second réservoir (16) en communication électrique l'un avec l'autre, et dans lequel (a) ledit premier moyen de support (20) et ledit second moyen de support (22) sont raccordés par charnière le long d'un élément pouvant être courbé (34) de façon que le dispositif (10) puisse être activé en repliant les moyens de support (20, 22) le long de l'élément pouvant être courbé (34) pour mettre le premier réservoir (14) et le second réservoir (16) en communication électrique, ou (b) le dispositif comprend un moyen de barrière (240) pour séparer ledit premier réservoir dudit second réservoir, qui peut être manipulé pour mettre le premier réservoir et le second réservoir en contact électrique conducteur l'un avec l'autre ; et
ledit moyen de barrière (240) est adapté pour inclure une surface supérieure détachable (242), une surface inférieure détachable (244) et une languette (246) s'étendant à partir du moyen de support de sorte que le dispositif puisse être activé en tirant la languette (246) pour enlever la barrière (240) du dispositif et mettre le premier réservoir et le second réservoir en communication électrique.

2. Dispositif activé par un utilisateur selon la revendication 1, dans lequel ledit premier réservoir inclut un second médicament à délivrer à l'endroit d'application du patient, et ledit second réservoir est maintenu dans un état sec avant l'activation.

3. Dispositif activé par un utilisateur selon l'une ou l'autre des revendications précédentes, comprenant en outre au moins une barrière (38) située entre le premier réservoir et le second réservoir pour limiter la présence d'ions concurrents lorsque les deux réservoirs sont en contact électrique conducteur l'un avec l'autre.

4. Dispositif activé par un utilisateur selon la revendication 1, qui est un dispositif iontophorétique dans lequel ledit second réservoir contient un médicament de type agent vasoactif substantiellement non hydraté à délivrer à l'endroit d'application du patient.

5. Dispositif iontophorétique activé par un utilisateur selon la revendication 4, dans lequel le premier réservoir inclut un anesthésique local et l'agent vasoactif est un composé vasoconstricteur.

6. Dispositif iontophorétique activé par un utilisateur selon la revendication 5, dans lequel l'anesthésique local est la lidocaine et le composé vasoconstricteur est l'adrénaline.

7. Dispositif activé par un utilisateur selon l'une quelconque des revendications précédentes, dans lequel l'électrolyte est un gel électroconducteur.
